# EUROPEAN PATENT SPECIFICATION

(11) **EP 3 524 289 B1**
(45) Date of publication and mention of the grant of the patent: **10.03.2021**
(21) Application number: 19161541.8
(22) Date of filing: 25.12.2015
(51) Int. Cl.: A61M 1/34, A61M 1/14

(54) **BLOOD TREATMENT SYSTEM**
BLUTBEHANDLUNGSSYSTEM
SYSTÈME DE TRAITEMENT DU SANG

(30) Priority: 25.12.2014 JP 2014262923
(43) Date of publication of application: 14.08.2019
(62) Divisional of application: 15873288.3
(73) Proprietor: Asahi Kasei Medical Co., Ltd., Tokyo 100-0006 (JP)
(72) Inventor: Kouda, Masaaki, Tokyo, 101-8101 (JP)
(74) Representative: dompatent von Kreisler Selting Werner - Partnerschaft von Patent- und Rechtsanwälten mbB

(56) References cited:
- WO-A1-93/01858
- JP-A- H09 239 024
- US-A- 5 665 061

## Description

### Technical Field

The present invention is related to a blood treatment system.

### Background Art

An example of a therapeutic method for severely ill patients with, for example, acute renal failure, etc. includes continuous hemofiltration therapy which continuously purifies blood through extracorporeal circulation. This treatment is performed using a blood treatment device, and the blood treatment device has a blood treatment circuit that supplies blood of a patient to a blood purification unit and returns the blood to the patient after purification, and a therapeutic fluid supply circuit that supplies the therapeutic fluid to be used in the blood treatment circuit to the blood treatment circuit. The therapeutic fluid includes fluid replacement that is supplied to the blood in the blood treatment circuit for maintaining the body fluid balance of a patient, and a dialysate supplied to the blood purification unit for removing waste product from the patient's blood. The therapeutic fluid is stored in a reservoir of a therapeutic fluid supply circuit and is supplied to the blood treatment circuit from the reservoir by a supply pump. An example of a blood purification system is disclosed in JPH09239024.

### Citation List

### Patent Document

Patent Document 1: JP4458346

### Summary

### Technical Problem

The supply flow rate of the above-mentioned therapeutic fluid needs to be strictly controlled as it affects the body fluid amount and body fluid component of a patient. Hence, consideration is made on providing a weight scale in the reservoir in the blood treatment device so as to calculate the actual flow rate of the supply pump from the variation in the weight of the reservoir measured by the weight scale, to thereby strictly control the supply pump from said actual flow rate.

Since blood is treated continuously for a long time in the blood treatment device in a continuous hemofiltration therapy, it is preferable for the therapeutic fluid which includes the dialysate and fluid replacement used in said blood treatment to be able to be refilled in the reservoir of the blood treatment device. Hence, it has been proposed to connect the dialysate preparing device to the blood treatment device, and to produce a dialysate using said dialysate preparing device to supply said dialysate to the blood purification device (see Patent Document 1).

However, when attempting to refill the therapeutic fluid into the reservoir during blood treatment using the above-mentioned dialysate preparing device, the weight of the reservoir varies due to the therapeutic fluid supplied from elsewhere and thus the above-mentioned control of the flow rate of a supply pump based on the weight of the reservoir will not be able to be performed. Therefore, refilling the therapeutic fluid into the reservoir while performing blood treatment is difficult in practice.

The present invention has been made by taking the above points into consideration, and the present invention is intended to provide a blood treatment system capable of strictly controlling the supply flow rate of a therapeutic fluid to a blood treatment circuit while refilling the therapeutic fluid into a reservoir of a blood treatment device during blood treatment.

### Solution to Problem

As a result of dedicated research by the inventors to achieve the above object, the inventors have found that, by providing a weight scale to each of the reservoir of the therapeutic fluid supply circuit and the reservoir of the therapeutic fluid refill circuit and controlling the supply pump based on the variation in the sum of the weight of the reservoir of the therapeutic fluid supply circuit and the weight of the reservoir of the therapeutic fluid refill circuit, the supply flow rate of the therapeutic fluid to the blood treatment circuit may be strictly controlled while refilling the therapeutic fluid into the reservoir of the blood treatment device during blood treatment. Hence the present invention has been achieved. More specifically, the following (1)-(7) will be provided as an example of the present invention.
(1) A blood purification system comprising: a blood treatment circuit which comprises a blood purification unit, supplies blood to the blood purification unit, and recovers purified blood from the blood purification unit; a therapeutic fluid supply circuit that supplies a therapeutic fluid used in the blood treatment circuit that is stored in a first reservoir to the blood treatment circuit by a supply pump; a therapeutic fluid refill circuit that refills therapeutic fluid stored in a second reservoir into the first reservoir; a first weight scale that measures a weight of the first reservoir; a second weight scale that measures a weight of the second reservoir; and a controller that calculates an actual flow rate of the supply pump from a variation in a sum of a weight of the first reservoir measured by the first weight scale and a weight of the second reservoir measured by the second weight scale and controls the supply pump based on the actual flow rate.
(2) The blood purification system according to (1), wherein, in a state in which a new therapeutic fluid is not introduced from outside into the therapeutic fluid refill circuit and the therapeutic fluid supply circuit, the controller calculates an actual flow rate of the supply pump from a variation in a sum of a weight of the first reservoir measured by the first weight scale and a weight of the second reservoir measured by the second weight scale.
(3) The blood purification system according to (1) or (2), wherein the controller calculates a fluid rate error between the actual flow rate and a target flow rate of the supply pump, and corrects the flow rate error.
(4) The blood purification system according to any one of (1) to (3) further comprising: a drainage circuit that discharges drainage occurring in the blood purification unit into a drainage reservoir by a drainage pump; an external drainage circuit that discharges, to the outside, drainage stored in the drainage reservoir; a third weight scale that measures a weight of the drainage reservoir; and a drainage controller that calculates, when the drainage of the drainage reservoir is not discharged to the outside from the external drainage circuit, an actual flow rate of the drainage pump from a variation in a weight of the drainage reservoir measured by the third weight scale, and corrects a flow rate error between the actual flow rate and the target flow rate by controlling the drainage pump, and controls, when the drainage of the drainage reservoir is discharged to the outside from the external drainage circuit, the drainage pump using a corrected value of the flow rate error from immediately before initiating the discharging to the outside.
(5) The blood purification system according to any one of (1) to (4) comprising a plurality of the second reservoirs, wherein the second weight scales are provided for each of the second reservoirs and the therapeutic fluid is refilled into the first reservoir from one second reservoir selected sequentially from among the plurality of second reservoirs, wherein the controller calculates an actual flow rate of the supply pump from a variation in a sum of a weight of the second reservoir which refills the therapeutic fluid into the first reservoir and a weight of the first reservoir, and controls the supply pump based on the actual flow rate.
(6) The blood purification system according to any one of (1) to (5) further comprising: a therapeutic fluid generating section that generates a therapeutic fluid; and a therapeutic fluid circuit that supplies the therapeutic fluid generated in the therapeutic fluid generating section to the second reservoir so as to store the therapeutic fluid.
(7) A blood purification system comprising: a blood treatment circuit that supplies blood to a blood purification unit and recovers purified blood from the blood purification unit; a drainage circuit that discharges drainage occurring in the blood purification unit to a drainage reservoir by a drainage pump; an external drainage circuit that discharges, to the outside, drainage stored in the drainage reservoir; a weight scale that measures a weight of the drainage reservoir; and a drainage controller that calculates, when the drainage of the drainage reservoir is not discharged to the outside from the external drainage circuit, an actual flow rate of the drainage pump from a variation of a weight of the drainage reservoir measured by the weight scale, and corrects a flow rate error between the actual flow rate and a target flow rate by controlling the drainage pump, and controls, when the drainage of the drainage reservoir is discharged to the outside from the external drainage circuit, the drainage pump using a corrected value of the flow rate error from immediately before initiating the discharging of the drainage.

### Advantageous Effects of Invention

Since the present invention is able to strictly control the supply flow rate of a therapeutic fluid to a blood treatment circuit while refilling the therapeutic fluid into a reservoir of a blood treatment device during blood treatment, it will be possible to actualize prolonged blood treatment such as continuous blood purification treatment.

### Brief Description of Drawings

Fig. 1 is an explanatory diagram indicating the outline of the configuration of a blood purification system.
Fig. 2 is a graph indicating a variation over time of an actual flow rate of a tube pump of a therapeutic fluid supply circuit.
Fig. 3 is a graph indicating a variation over time of an actual flow rate of a tube pump of a drainage circuit.
Fig. 4 is an explanatory diagram indicating a configuration of a blood treatment system having an external drainage circuit.
Fig. 5 is a graph for explaining the control of a tube pump of a drainage circuit upon discharging drainage in the drainage reservoir.
Fig. 6 is an explanatory diagram indicating a configuration of a blood treatment system having two second reservoirs.

### Description of Embodiments

The invention is described by the appended claims. The same reference sign will be applied to the same element and overlapping explanations will be omitted. Further, unless otherwise mentioned, the positional relationship in the drawings, such as top, bottom, left, and right, etc., will be based on the positional relationships indicated in the drawings. Moreover, the dimensional ratios of the drawings are not limited to the illustrated ratios.

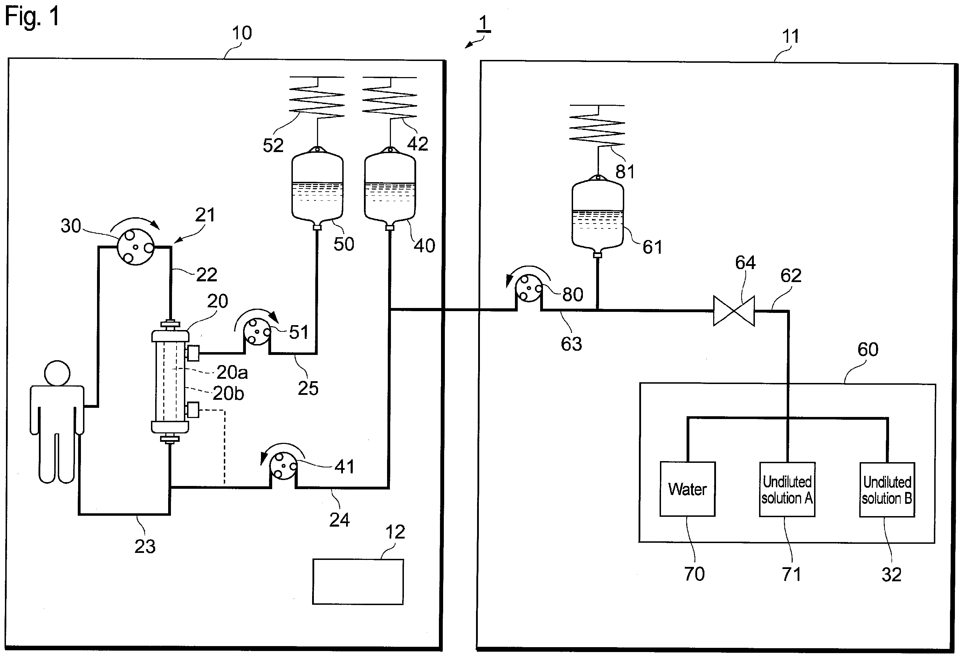

Fig. 1 is an explanatory diagram indicating the outline of the configuration of the blood treatment system 1 according to the present embodiment. The blood treatment system 1 is for performing, for example, a continuous blood purification treatment and the blood treatment system 1 comprises a blood treatment device 10 for performing blood treatment on a patient, a therapeutic fluid supply device 11 for supplying and refilling therapeutic fluid which includes dialysate and fluid replacement to the blood treatment device 10, and a controller 12 (including a drainage control device).

The blood treatment device 10 comprises a blood treatment circuit 21 that supplies blood of a patient to the blood purification unit 20 and returns said blood to the patient. The blood treatment circuit 21 comprises a blood supply circuit 22 for supplying blood of a patient to the blood purification unit 20, and a blood return circuit 23 for returning blood from the blood purification unit 20 to the patient.

Further, the blood treatment device 10 comprises a therapeutic fluid supply circuit 24 which performs either at least supplying of fluid replacement to the blood supply circuit 22 and/or blood return circuit 23 or supplying of dialysate to the blood purification unit 20, and a drainage circuit 25 for discharging drainage generated in the blood purification unit 20.

The blood purification unit 20 is, for example, a hollow fiber module including a hollow fiber membrane, wherein the blood purification unit 20 is able to cause the waste product, etc. within the blood supplied to the primary side 20a of the hollow fiber membrane to pass through the secondary side 20b of the hollow fiber membrane so as to filter and purify the blood.

The blood supply circuit 22 is, for example, connected to an inlet of the primary side 20a of the blood purification unit 20 from a needle portion (not illustrated) injected into a patient. A pump 30 is provided in the blood supply circuit 22 and the patient's blood may be supplied to the blood purification unit 20 at a predetermined flow rate. As the pump 30, for example, a so-called tube pump which sends fluid within the tube by pushing the tube configuring the circuit, is used.

The blood return circuit 23 is connected to the needle part (not illustrated) from an outlet of the primary side 20a of the blood purification unit 20.

The therapeutic fluid supply circuit 24 is connected to either the blood supply circuit 22 and/or blood return circuit 23 or the secondary side 20b of the blood purification unit 20 from the first reservoir 40. It can be selected as to whether the solution supply circuit 24 is connected to the blood supply circuit 22 and/or blood return circuit 23, connected to the blood purification unit 20, or connected to a plurality thereof according to the type of blood purification treatment which includes, for example, Continuous HemoFiltration (CHF), Continuous HemoDialysis (CHD), and Continuous HemoDiaFiltration (CHDF).

A tube pump 41 is provided as a supply pump in the therapeutic fluid supply circuit 24 and the therapeutic fluid, which includes dialysate and fluid replacement, of the first reservoir 40 is able to be supplied at a predetermined flow rate. The first reservoir 40 is provided with a first weight scale 42 that measures the weight of said reservoir 40.

The drainage circuit 25 is connected to the drainage reservoir 50 from the secondary side 20b of the blood purification unit 20. A tube pump 51 is provided in the drainage circuit 25 as a drainage pump, and the drainage of the blood purification unit 20 may be discharged into the drainage reservoir 50 at a predetermined flow rate. The drainage reservoir 50 is provided with a third weight scale 52 that measures the weight of said reservoir 50.

The therapeutic fluid supply device 11 comprises: a therapeutic fluid generating section 60 that generates the therapeutic fluid; a therapeutic fluid supply circuit 62 for supplying the therapeutic fluid generated in the therapeutic fluid generating section 60 to the second reservoir 61; and a therapeutic fluid refill circuit 63 for refilling the therapeutic fluid of the second reservoir 61 into the first reservoir 40 of the blood treatment device 10.

The therapeutic fluid generating section 60 comprises, for example, a water supply source 70, an undiluted solution A supply source 71, and an undiluted solution B supply source 72, and the therapeutic fluid generating section 60 is able to generate a therapeutic fluid by mixing these fluids at a predetermined ratio. As the water for the water supply source 70, for example, reverse osmosis filtered water and ultrafiltered water, etc. may be used. For example, an undiluted solution of a solution not including sodium bicarbonate may be used for the undiluted solution A and, for example, an undiluted solution of a solution including sodium bicarbonate may be used for the undiluted solution B.

The therapeutic fluid supply circuit 62 is connected to the second reservoir 61 from the therapeutic fluid generating section 60, and the therapeutic fluid refill circuit 63 is connected to the therapeutic fluid supply circuit 24 of the blood treatment device 10 from the second reservoir 61. The therapeutic fluid supply circuit 62 is provided with a therapeutic fluid closing means 64, such as an on-off valve, and a tube pump. The therapeutic fluid refill circuit 63 is provided with a tube pump 80 and the therapeutic fluid of the second reservoir 61 may be supplied to the therapeutic fluid supply circuit 24 at a predetermined flow rate. The second reservoir 61 is provided with a second weight scale 81 that measures the weight of the reservoir 61.

The controller 12 is a computer that controls the operation of the entire blood treatment system 1 and it is able to perform blood treatment and refilling of therapeutic liquids by controlling the operation of the tube pumps 30, 41, 51, and 80, and the therapeutic liquid closing means 64, etc. For example, the controller 12 comprises a program that corrects flow rate error of the tube pump 41 based on the measurement results measured by the weight scales 42, 81, and controls the flow rate of the therapeutic fluid supplied to the blood treatment circuit 21, as mentioned below. The blood treatment device 10 and the therapeutic fluid supply device 11 each comprise a control computer and the controller 12 may control the whole system through wired communication or wireless communication.

Next, the operation of the blood treatment system 1 configured as set forth above will be described.

A continuous blood purification treatment for a patient is performed in the blood treatment device 10 indicated in Fig. 1. For example, when Continuous HemoFiltration (CHF) therapy is performed, the patient's blood is supplied to the blood purification unit 20 via the blood supply circuit 22 by the tube pump 30. The waste product, etc. removed through a membrane from the blood in the blood purification unit 20 is discharged into the drainage reservoir 50 via the drainage circuit 25 by the tube pump 51. Further, the fluid replacement of the first reservoir 40 is supplied to the blood supply circuit 22 and/or blood return circuit 23 via the therapeutic fluid supply circuit 24 by the tube pump 41. The blood purified by the blood purification unit 20 is returned to the patient via the blood return circuit 23. Further when Continuous HemoDialysis (CHD) therapy is performed, the dialysate of the first reservoir 40 is supplied to the secondary side 20b of the membrane of the blood purification unit 20 by the tube pump 41. When Continuous HemoDiaFiltration (CHDF) therapy is performed, the fluid replacement is supplied to the blood supply circuit 22 and/or blood return circuit 23 and the dialysate is supplied to the blood purification unit 20 by the tube pump 41.

Further, in the therapeutic fluid supply device 11, while the blood purification treatment is performed in the blood treatment device 10, the therapeutic fluid in the second reservoir 61 is supplied to and refilled in the first reservoir 40 of the blood treatment device 10, as appropriate. Upon doing so, the therapeutic fluid is supplied to the therapeutic fluid supply circuit 24 of the blood treatment device 10 via the therapeutic fluid refill circuit 63 by the tube pump 80. The therapeutic fluid supplied to the therapeutic fluid supply circuit 24 is supplied to and stored mainly in the first reservoir 40 and, in some cases, some is directly supplied to at least one of the blood supply circuit 22, the blood return circuit 23, or the blood purification unit 20. It should be noted that the therapeutic fluid is generated in the therapeutic fluid generating section 60 during the stopping of the supply of therapeutic fluid and that the therapeutic fluid is stored in the second reservoir 61 in advance. The therapeutic fluid supply closing means 64 is closed during the supply of the therapeutic fluid and the therapeutic fluid will not be refilled into the second reservoir 61. It should be noted that, for example, as the means for supplying the therapeutic fluid from the second reservoir 61 to the first reservoir 40, liquid feeding using a valve and a head of fluid can be considered, instead of the tube pump 80. However, in a case of the method of supply using the head of fluid, when the tube pump 41 is actuated at a high flow rate, the refill of the therapeutic fluid by the head of fluid will be too slow and thus the therapeutic fluid within the first reservoir 40 will be emptied quickly, thereby resulting in not being able to perform a stable supply of the therapeutic fluid. In addition, the use of the head of fluid and the valve is space consuming, and an increase in the size of the entire blood treatment system 1 is therefore inevitable. Hence, it is preferable to use a tube pump 80.

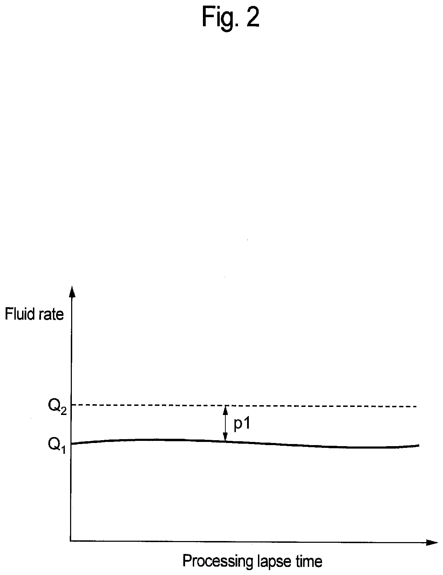

During the blood purification treatment, the flow rate of the supply of the therapeutic fluid to the blood treatment circuit 21 is strictly controlled by the controller 12. For example, during the blood purification treatment, the weight of the first reservoir 40 may be continuously measured by the first weight scale 42 and the weight of the second reservoir 61 may be continuously measured by the second weight scale 81. These measurement results are output into the controller 12 and in the controller 12, and the actual flow rate Q1 of the therapeutic fluid supplied by the tube pump 41 is calculated from the variation in the sum of the weight of the first reservoir 40 and the weight of the second reservoir 61. For example, the actual flow rate Q1 may be calculated with the formula Q1=ΔM/measurement time from the variation ΔM of the sum M of the weight of the first reservoir 40 and the weight of the second reservoir 61. Next, the controller 12 corrects the flow rate error p1 (Q2-Q1) between the actual flow rate Q1 of the tube pump 41 and the target flow rate Q2 set in advance as indicated in, for example, Fig. 2. Specifically, for example, the flow rate error p1 may be corrected by increasing the output of the tube pump 41 only by the amount of the flow rate error. The controller 12 performs the feedback control of this tube pump 41 continuously or intermittently, and strictly controls the flow rate of the therapeutic liquid supplied to the blood treatment circuit 21.

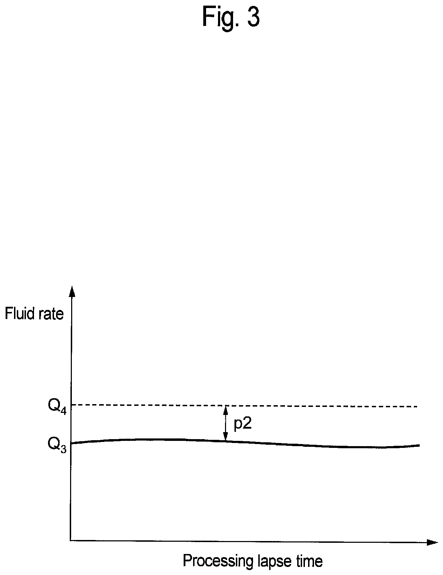

Further, during the blood purification treatment, the flow rate of the drainage from the blood purification unit 20 of the blood treatment circuit 21 to the drainage reservoir 50 is strictly controlled by the controller 12. For example, during the blood purification treatment, the weight of the drainage reservoir 50 may be continuously measured by the third weight scale 52. This measurement result is output to the controller 12 and the actual flow rate Q3 of the drainage discharged by the tube pump 51 is calculated from the variation of the weight of the drainage reservoir 50 in the controller 12. Next, the controller 12 corrects the flow rate error P2 (Q4-Q3) of the actual flow rate Q3 of the tube pump 51 and the target flow rate Q4 set in advance, as indicated in, for example, Fig. 3. Specifically, for example, the flow rate error p2 may be corrected by increasing the output of the tube pump 51 only by the amount of the flow rate error. The controller 12 performs the feedback control of this tube pump 51 continuously or intermittently, and strictly controls the flow rate of the drainage discharged from the blood purification unit 20.

According to the present embodiment, the weight of the first reservoir 40 and the weight of the second reservoir 61 are measured and the actual flow rate Q1 of the tube pump 41 is calculated from the variant ΔM of the sum of such measurement, and thus the flow rate error p1 between the actual flow rate Q1 and the target flow rate Q2 is corrected by controlling the tube pump 41. Hence, even if the therapeutic fluid is refilled into the first reservoir 40 of the blood treatment device 10 during the blood treatment, the flow rate control of the tube pump 41 based on the decrease in the therapeutic fluid of the reservoir may still be performed. Therefore, the supply flow rate of the therapeutic fluid to the blood treatment circuit 21 may be strictly controlled by refilling the therapeutic fluid into the first reservoir 40 of the blood treatment device 10 during the blood treatment.

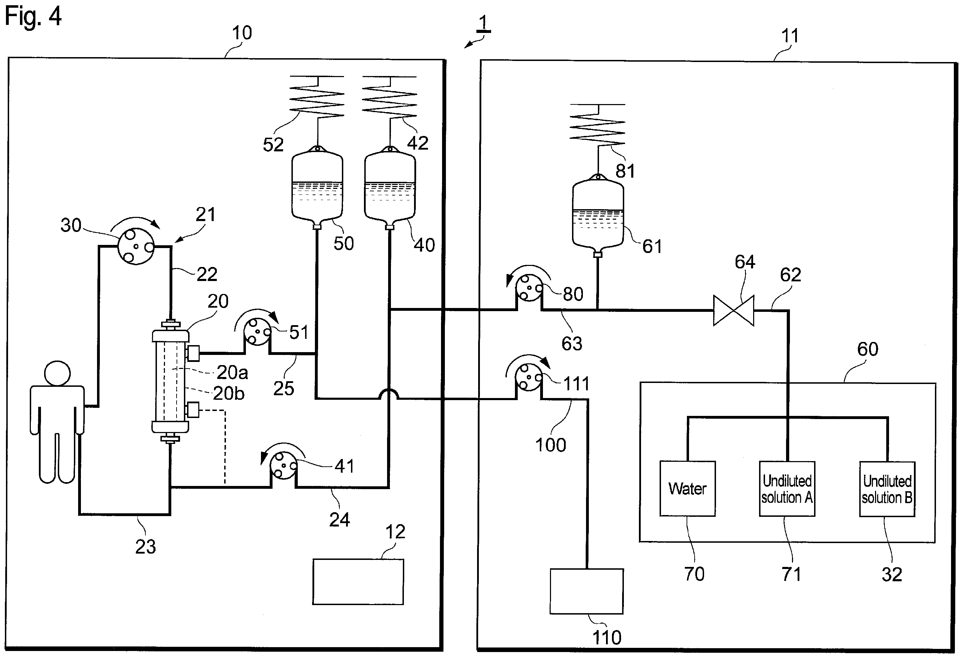

In the above-mentioned embodiment, as indicated in Fig. 4, the blood treatment system 1 may further comprise an external drainage circuit 100 that discharges the drainage stored in the drainage reservoir 50 to the outside so as to have the drainage of the drainage reservoir 50 discharged to the outside as appropriate during the blood treatment.

In such a case, for example, the external drainage circuit 100 may be connected to the drainage section 110 of the therapeutic fluid supply device 11 from the drainage circuit 25 and may comprise a tube pump 111. Further, when a drainage exceeding the predetermined threshold is pooled in the drainage reservoir 50 during blood treatment, the tube pump 111 is actuated and the drainage of the drainage reservoir 50 is discharged into the drainage section 110 via the external drainage circuit 100. As the blood treatment is continued upon doing so, the tube pump 51 of the drainage circuit 25 remains in operation. Further, when the drainage in the drainage reservoir 50 is decreased to below the predetermined amount, the tube pump 111 is stopped and the discharge of the drainage is stopped.

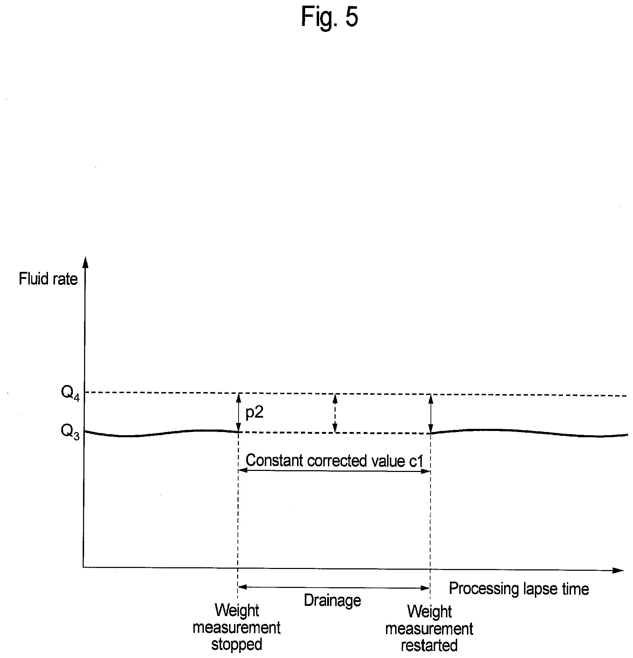

The flow rate control of the tube pump 51 of the drainage circuit 25 at this time is performed as follows. As mentioned above, when the drainage of the drainage reservoir 50 is not discharged via the external drainage circuit 100, the actual flow rate Q3 of the tube pump 51 is calculated from the variation of the weight of the drainage reservoir 50 measured by the third weight scale 52, and the flow rate error p2 between the actual flow rate Q3 and the target flow rate Q4 is corrected by controlling the tube pump 51. Moreover, while the drainage of the drainage vessel 50 is discharged via the external drainage circuit 100, as indicated in Fig. 5, the tube pump 51 is controlled utilizing the corrected value of the flow rate error p2 from immediately before initiating the discharge to the outside. In other words, when the corrected value of the flow rate error p2 from immediately before initiating the discharge of the drainage of the drainage reservoir 50 is c1, the corrected value c1 of the flow rate error thereof will be maintained during drainage. The measurement of the weight of the drainage reservoir 50 by the third weight scale 52 is stopped during the discharge of the drainage. Further, when the drainage of the drainage reservoir 50 is finished, the measurement of the weight of the drainage reservoir 50 by the third weight scale 52 is initiated again and the actual flow rate Q3 of the tube pump 51 is calculated based on said weight and thus the flow rate error p2 between the actual flow rate Q3 and the target flow rate Q4 is corrected by controlling the tube pump 51.

According to this embodiment, since the drainage of the drainage reservoir 50 may be discharged during the blood treatment, the blood treatment can be performed continuously for a prolonged time. Further, the actual flow rate Q3 of the tube pump 51 will be stabilized and the corrected value c1 of the flow rate error p2 thereof will be stabilized when the blood treatment is continued for a long time until the drainage is pooled sufficiently in the drainage reservoir 50. Hence, the control of the tube pump 51 during the drainage is performed using the corrected value c1 of the flow rate error p2 from immediately before initiating the discharge of the drainage. By achieving this, the flow rate of the drainage from the blood purification unit 20 may be strictly controlled while performing the discharge of the drainage during the blood treatment. Therefore, the prolonged blood treatment by the blood purification unit 20 will be performed appropriately.

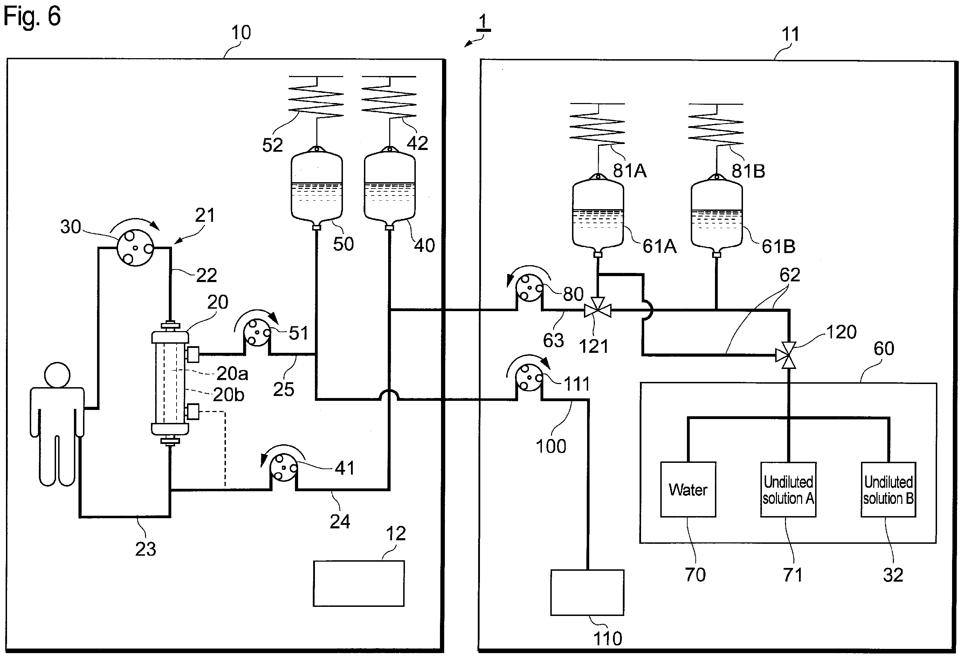

Next, in the above embodiment, as indicated in Fig. 6, the blood treatment system 1 may comprise a plurality of the second reservoirs 61 (for example, two), have the second weight scales 81A, 81B provided for each of said second reservoirs 61A, 61B, and have the therapeutic fluid refilled into the first reservoir 40 from one second reservoir 61A, 61B selected in sequential order from among a plurality of the second reservoirs 61A, 61 B.

In such case, the therapeutic liquid supply circuit 62 is branched on its way so that it will be connected respectively to each of the second reservoirs 61A, 61B. In such branch point, for example, a three-way valve 120 may be provided. Further, the therapeutic liquid supply circuit 63 is branched on its way so that it will be connected respectively to each of the second reservoirs 61A, 61B. For example, a three-way valve 121 may be provided to this branching point.

During the blood treatment, either of the second reservoirs 61A, 61B is selected as the source of the therapeutic fluid to be refilled into the first reservoir 40, and the therapeutic fluid from the therapeutic fluid generating section 60 is refilled into the second reservoir 61A, 61B that is not selected. For example, when the second reservoir 61A is selected, the therapeutic fluid may be refilled into the first reservoir 40 from the second reservoir 61A upon the refilling of the therapeutic fluid and the therapeutic fluid from the therapeutic fluid generating section 60 may be refilled into the second reservoir 61B, as appropriate. Further, when the second reservoir 61B is being selected, the therapeutic fluid is refilled into the first reservoir 40 from the second reservoir 61 B upon the refilling of the therapeutic fluid and the therapeutic fluid from the therapeutic fluid generating section 60 is refilled into the second reservoir 61A, as appropriate. The selection of this second reservoir 61A and the second reservoir 61B are performed in an alternating manner.

Further, the fluid rate control of the tube pump 41 of the therapeutic fluid supply circuit 24 calculates the actual flow rate Q1 of the tube pump 41 from the variation of the sum of one of the (selected) second reservoir 61 which is refilling the therapeutic fluid into the first reservoir 40 and the sum of the first reservoir 40, and the flow rate error p1 between the actual flow rate Q1 and the target flow rate Q2 is corrected by controlling the tube pump 41.

According to this embodiment, since the blood treatment system 1 comprises a plurality of second reservoirs 61 and is capable of alternately performing the refilling of the therapeutic fluid into the first reservoir 40 and the refilling of the therapeutic fluid into the second reservoir 61, it is possible to reduce the size of the pump as the flow rate of the tube pump 80 may be equal to or greater than the flow rate needed for the tube pump 41. Further, the supply flow rate of the therapeutic fluid of the tube pump 41 at such time may be strictly controlled. It should be noted that the number of the second reservoirs 61 in the present embodiment is not limited to two and, thus, any number can be selected. Here, although the blood treatment system 1 is configured from the blood treatment device 10 and the therapeutic fluid supply device 11, the two devices may be either independently configured or integrated. However, according to the embodiment of the therapy, the therapy may be implemented without utilizing the therapeutic fluid supply device 11 by supplying the therapeutic fluid not from the therapeutic fluid supply device 11 but connecting the prepared known therapeutic fluid bag in place of the first reservoir 40. Hence, in order to allow flexibility in the embodiment of the therapy, it is preferable to provide the first reservoir 40 at the side of the blood treatment device 10 and to provide the second reservoir 61 at the side of the therapeutic fluid supply device 11 and to configure the blood treatment device 10 and the therapeutic fluid supply device 11 separately. Further, the blood treatment device 10 already exists in the market and thus heavy investment will be necessary upon developing an integrated configuration. Hence, by designing the blood treatment device 10 and the therapeutic fluid supply device 11 as independent devices, along with being able to keep development costs low, smooth introduction into the known market will be possible. When the blood treatment device 10 and the therapeutic fluid supply device 11 are configured separately, the circuits of the respective devices are connected by communicating the therapeutic fluid refill circuit 63 and the therapeutic fluid supply circuit 24. For example, a luer connector, etc. may be used for connection but such connection will not be limited thereto, and any connection may be used as long as it is capable of connecting the respective circuits. Further, with respect to the connection position, although the therapeutic fluid refill circuit 63 and the therapeutic fluid supply circuit 24 may be directly connected or may be connected via the first reservoir 40, the position of connection will not be limited thereto, and any position of connection may be selected as long as the therapeutic fluid is able to be supplied to the first reservoir 40. Moreover, when the therapeutic refill circuit 63 is not connected to the connection for the therapeutic fluid supply circuit 24 in advance, a clamp, a check valve and a needleless mixed injection tube, etc. may be arranged at the connection in order to prevent leakage of the therapeutic fluid. Further, when the blood treatment device 10 and the therapeutic supply device 11 are configured separately, it is necessary to perform control by alternately sharing the measurement results of the first weight scale 42 and the second weight scale 81. In order to achieve this, it is necessary to have the blood treatment device 10 and the therapeutic fluid supply device 11 be electrically linked and, as the means thereof, physical connections such as RS232 cable, RS422 cable, LAN cable and USB cable, and a wireless signal such as Bluetooth (registered trademark) and infrared light may be listed as examples. However, such connection is not limited thereto, and any connection may be selected as long as information necessary for control can be transmitted.

Although the preferable embodiment of the present invention has been explained above by referring to the attached drawings, the present invention is not limited to such example. It is obvious that a person skilled in the art will be able to think of various modifications and corrections within the scope of the idea recited in the scope of claims and it is naturally understood that such modifications and corrections belong to the technical scope of the present invention.

For example, although the second reservoir 61 was at the side of the therapeutic fluid supply device 11 in the above-mentioned embodiment, the embodiment is not limited to such arrangement and thus the second reservoir 61 may be at the side of the blood treatment device 10. The circuit configuration of the blood treatment system 1 is not limited to such configuration. The controlled pumps 41, 51 do not have to be tube pumps.

In the above-mentioned embodiment, although the fluid rate control of the tube pump 51 of the drainage circuit 25 was performed on top of performing the flow rate control of the tube pump 41 of the therapeutic fluid supply circuit 24, it is possible for only the flow rate control of the tube pump 51 to be performed. In other words, while the drainage of the drainage reservoir 50 is not discharged to the outside via the external drainage circuit 100, the actual flow rate Q3 of the tube pump 51 may be calculated from the variation in the weight of the drainage reservoir 50 measured by the weight scale 52, and the flow rate error p2 between the actual flow rate Q3 and the target flow rate Q4 may be corrected by controlling the tube pump 51, and while the drainage of the drainage reservoir 50 is discharged outside via the external drainage circuit 100, the tube pump 51 may be controlled using the corrected value c1 of the flow rate error p2 from immediately before initiating the discharge of said drainage. Furthermore, the blood treatment system of the present invention may be applied to a case in which the therapeutic fluid is supplied to blood treatment devices other than continuous blood treatment devices.

### Industrial Applicability

The present invention is effective upon providing a blood treatment system that strictly controls the supply flow rate of a therapeutic fluid to a blood treatment circuit while refilling the therapeutic fluid into a reservoir of a blood treatment device during blood treatment.

### Reference Signs List

1 Blood treatment system
10 Blood treatment device
11 Therapeutic fluid supply device
12 Control device
20 Blood purification unit
21 Blood treatment circuit
40 First reservoir
42 First weight scale
41 Tube pump
50 Drainage reservoir
51 Tube pump
52 Third weight scale
61 Second reservoir
81 Second weight scale

## Claims

1. A blood purification system (1) comprising:
a blood treatment circuit (21) configured to supply blood to a blood purification unit (20) and recovers purified blood from the blood purification unit (20);
a drainage circuit (25) configured to discharge drainage occurring in the blood purification unit (20) to a drainage reservoir (50) by a first drainage pump (51);
an external drainage circuit (100) configured to discharge, to outside, by a second drainage pump (111), drainage stored in the drainage reservoir (50);
a weight scale (52) configured to measure the weight of the drainage reservoir (50); and
a drainage controller (12) configured to calculate, when drainage of the drainage reservoir (50) is not discharged to the outside from the external drainage circuit (100), an actual flow rate of the first drainage pump (51) from a variation of a weight of the drainage reservoir (50) measured by the weight scale (52) and configured to correct a flow rate error between the actual flow rate and a target flow rate by controlling the first drainage pump (51), and configured to control, when drainage of the drainage reservoir (50) is discharged to the outside from the external drainage circuit (100) by the second drainage pump (111), the first drainage pump (51) using a corrected value of the flow rate error from immediately before initiating the discharging of drainage,
wherein the second drainage pump (111) is configured such that when a drainage exceeding a predetermined threshold is pooled in the drainage reservoir (50), the second drainage pump (111) is actuated, and when the drainage in the drainage reservoir (50) is decreased to below the predetermined amount, the second drainage pump (111) is stopped.

## Patentansprüche

1. Blutreinigungssystem (1), umfassend:
einen Blutbehandlungskreislauf (21), der so konfiguriert ist, dass er einer Blutreinigungseinheit (20) Blut zuführen kann, und gereinigtes Blut aus der Blutreinigungseinheit (20) zurückgewinnt;
einen Ableitkreislauf (25), der so konfiguriert ist, dass er abzuleitende Flüssigkeit, die in der Blutreinigungseinheit (20) auftritt, durch eine erste Ableitpumpe (51) in ein Ableitreservoir (50) ausleiten kann;
einen externen Ableitkreislauf (100), der so konfiguriert ist, dass er in dem Ableitreservoir (50) gelagerte abzuleitende Flüssigkeit durch eine zweite Ableitpumpe (111) nach außen ausleiten kann;
eine Waage (52), die so konfiguriert ist, dass sie das Gewicht des Ableitreservoirs (50) messen kann; und
ein Ableitungssteuergerät (12), das so konfiguriert ist, dass es, wenn keine abzuleitende Flüssigkeit aus dem Ableitreservoir (50) aus dem externen Ableitkreislauf (100) nach außen ausgeleitet wird, aus der Variation im durch die Waage (52) gemessenen Gewicht des Ableitreservoirs (50) die tatsächliche Fließgeschwindigkeit der ersten Ableitpumpe (51) berechnen kann, und so konfiguriert ist, dass es den Fließgeschwindigkeitsfehler zwischen der tatsächlichen Fließgeschwindigkeit und der Sollfließgeschwindigkeit durch Steuern der ersten Ableitpumpe (51) korrigieren kann, und so konfiguriert ist, dass es, wenn die abzuleitende Flüssigkeit aus dem Ableitreservoir (50) aus dem externen Ableitkreislauf (100) durch die zweite Ableitpumpe (111) nach außen ausgeleitet wird, die erste Ableitpumpe (51) mit Hilfe des korrigierten Werts des Fließgeschwindigkeitsfehlers gegenüber unmittelbar vor der Einleitung des Ausleitens der abzuleitenden Flüssigkeit steuern kann;
wobei die zweite Ableitpumpe (111) so konfiguriert ist, dass, wenn eine abzuleitende Flüssigkeit, die einen vorbestimmten Schwellenwert überschreitet, in dem Ableitreservoir (50) zusammengeführt wird, die zweite Ableitpumpe (111) betätigt wird, und wenn die abzuleitende Flüssigkeit in dem Ableitreservoir (50) unter die vorbestimmte Menge gesenkt wird, die zweite Ableitpumpe (111) angehalten wird.

## Revendications

1. Système de purification du sang (1) comprenant:
un circuit de traitement du sang (21) configuré pour fournir du sang à une unité de purification du sang (20) et récupère le sang purifié depuis l'unité de purification de sang (20);
un circuit de drainage (25) configuré pour évacuer le drainage se produisant dans l'unité de purification du sang (20) vers un réservoir de drainage (50) par une première pompe de drainage (51);
un circuit de drainage externe (100) configuré pour évacuer, vers l'extérieur, par une seconde pompe de drainage (111), le drainage stocké dans le réservoir de drainage (50);
une balance de poids (52) configurée pour mesurer le poids du réservoir de drainage (50); et
un organe de commande de drainage (12) configuré pour calculer, lorsque le drainage du réservoir de drainage (50) n'est pas évacué vers l'extérieur depuis le circuit de drainage externe (100), un débit réel de la première pompe de drainage (51) à partir d'une variation d'un poids du réservoir de drainage (50) mesuré par l'échelle de poids (52) et configuré pour corriger une erreur de débit entre le débit réel et un débit cible en commandant la première pompe de drainage (51), et configuré pour commander, lorsque le drainage du réservoir de drainage (50) est évacué vers l'extérieur depuis le circuit de drainage externe (100) par la seconde pompe de drainage (111), la première pompe de drainage (51) en utilisant une valeur corrigée de l'erreur de débit immédiatement avant le début de l'évacuation du drainage,
dans lequel la seconde pompe de drainage (111) est configurée de telle sorte que lorsqu'un drainage dépassant un seuil prédéterminé est rassemblé dans le réservoir de drainage (50), la seconde pompe de drainage (111) est actionnée, et lorsque le drainage dans le réservoir de drainage (50) est diminué jusqu'en dessous de la quantité prédéterminée, la seconde pompe de drainage (111) est arrêtée.
